# EUROPEAN PATENT SPECIFICATION

(11) **EP 1 802 366 B1**
(45) Date of publication and mention of the grant of the patent: **10.12.2008**
(21) Application number: 05787802.7
(22) Date of filing: 22.08.2005
(51) Int. Cl.: A61M 5/142, A61N 1/36, A61L 29/16, A61L 31/16, A61L 27/54

(54) **MEDICAL DEVICE WITH TISSUE-DEPENDENT DRUG ELUTION**
MEDIZINPRODUKT MIT GEWEBEABHÄNGIGER ARZNEIMITTELELUTION
DISPOSITIF MEDICAL A ELUTION DE MEDICAMENTS DEPENDANT DES TISSUS

(30) Priority: 20.08.2004 US 603488 P; 28.10.2004 US 975253
(43) Date of publication of application: 04.07.2007
(73) Proprietor: MEDTRONIC, INC., Minneapolis MN 55432-5604 (US)
(72) Inventor: LENT, Mark, S., Brooklyn Park, Minnesota 55433 (US); HERUTH, Kenneth, T., Edina, Minnesota 55424 (US)
(74) Representative: Hughes, Andrea Michelle
(86) International application number: PCT/US2005/029805
(87) International publication number: WO 2006/023859

(56) References cited:
- WO-A-01/41858
- WO-A-99/30772
- US-A1- 2002 193 879
- US-A1- 2004 034 337
- US-A1- 2004 158 313

## Description

### FIELD OF THE INVENTION

This invention relates to medical devices and systems having a polymeric coating as a vehicle for drug delivery, and more particularly to such devices and systems configured to have a portion be placed intravascularly or in the central nervous system (CNS) and a portion to be placed extravascularly or outside the CNS.

### BACKGROUND OF THE INVENTION

Implantation of medical devices, such as pacemakers, neurostimulators, implanted drug pumps, leads, catheters, etc, has been associated with adverse consequences, such as formation of scar tissue surrounding the implant, infection due to bacteria introduced during implantation, and tissue proliferation in blood vessels after a stent implantation. Attempts to prevent or control such adverse reactions have included administration of drugs, completely separate from the intended primary therapy of the implanted medical device. In some cases, systemically administered drugs, e.g. orally, intravenously, or intramuscularly administered drugs, have proven effective in treating complications due to medical device implantation. In other cases, systemic delivery has been ineffective due to, e.g., pharmacokinetic or pharmacodynamic characteristics of the drug, the location of the implanted device, or side effects of the drug. To increase effectiveness in these situations, some implanted devices have been modified to elute the drug into the surrounding tissues.

One common way of providing local drug elution is to dispose a polymer layer on the implantable medical device and embed the drug into the polymer during manufacturing. When hydrated after implant, the drug diffuses out of the polymer into surrounding tissue. Various methods of impregnating polymers with drugs have been used, including mixing the drug into the melted polymer prior to processing (e.g. molding or extrusion), and diffusing the drug into a finished polymer component using chemicals to swell the polymer for rapid loading. In some cases, the implantable medical device (IMD) is made from a polymer that is compatible with the drug, and the drug can be loaded directly into the device. However, many IMDs are made from metals or from polymers that are inherently incompatible with the desired drug. In such situations, the IMD can be coated with a thin layer of a compatible polymer, and the drug can be loaded into the coating layer.

WO 99/30772 discloses a subcutaneously implanted cardiac pulse generator having a lead with a tip coated in an anti-inflammatory compound. US 2004034357 discloses a stent having different portions coated with different therapeutic compositions. US 2004158313 discloses a stent with a narrow band of coating comprising a drug.

Some devices, such as catheters, leads, and lead extensions, which may be implanted throughout several different tissue locations of a patient, have been coated along their length with drug-containing polymeric materials, regardless of what tissue locations various portions of the device are implanted. By way of example, a catheter may be implanted in a patient such that a portion may be implanted in the patient's CNS and other portions of the catheter may be implanted subcutaneously. Coating devices in such a manner fails to take into consideration that the drug to be eluted from the polymeric coating may be efficacious in only one tissue or may produce side effects when eluted into another tissue.

### BRIEF SUMMARY OF THE INVENTION

In one embodiment, the invention provides an implantable medical catheter comprising an external surface, the external surface of the catheter comprising: (a) a first portion adapted to be implanted into a second tissue location; and a first therapeutic agent disposed on, in, or about at least a portion of the first portion but not the second portion.

In another embodiment of the invention, a first therapeutic agent is disposed on, in and/or about at least a portion of an exterior surface of the first portion of the device. A second therapeutic agent is disposed on, in, and/or about at least a portion of an exterior surface of the second portion of the device. The first and second therapeutic agents may be on or in a polymeric material.

One or more embodiments of the present invention may provide advantages over existing technology. For example, various embodiments of the invention target a therapeutic agent to a tissue location where its beneficial effects will be maximized. By disposing a therapeutic agent on, in or about a portion of a medical device lying in one tissue, but not another, allows for directed application of the agent to the tissue where its action is desired. Similarly, various embodiments of the invention prevent direct administration of a therapeutic agent to a tissue location where the agent may produce undesirable effects. Additional embodiments allow for the targeted delivery of a first therapeutic agent to a first tissue location and targeted delivery of a second therapeutic agent to a second tissue location, allowing for greater control of the desired and undesired effects of the agents to be delivered. These and other advantages will become evident upon reading the disclosure presented herein.

### BRIEF DESCRIPTION OF THE DRAWINGS

Figure 1 is a diagrammatic illustration of a device according to an embodiment of the invention.
Figure 2 is a diagrammatic illustration of a device implanted in two tissue locations according to an embodiment of the invention.
Figure 3 is a diagrammatic illustration of a device according to an embodiment of the invention.
Figure 4 is a diagrammatic illustration of a device implanted in two tissue locations according to an embodiment of the invention.
Figure 5 is a diagrammatic illustration of systems comprising an active device and an associated device according to embodiments of the invention.
Figure 6 is a diagrammatic illustration of a neurostimulator system, shown by way of background only.
Figure 7 is a diagrammatic illustration of an infusion delivery system according to an embodiment of the invention.
Figure 8 is a diagrammatic illustration of cross sections of other devices show by way of background only.
Figure 9 is a diagrammatic illustration of cross sections of devices according to embodiments of the invention.
Figure 10 is a diagrammatic illustration of cross sections of devices according to embodiments of the invention.
Figure 11 is a diagrammatic illustration of cross sections of devices according to embodiments of the invention
The drawings are not necessarily to scale. Like numbers refer to like parts or steps throughout the drawings.

### DETAILED DESCRIPTION OF THE INVENTION

In the following description, reference is made to the accompanying drawings that form a part hereof, and in which are shown by way of illustration several specific embodiments of the invention. It is to be understood that other embodiments of the present invention are contemplated and may be made without departing from the scope of the present invention. The following detailed description, therefore, is not to be taken in a limiting sense.

Various embodiments of the present invention relate to eluting therapeutic agents from a surface of a medical device. In particular embodiments, the invention provides systems and devices configured to have a first portion implanted in a first tissue and a second portion implanted in a second tissue. In some embodiments, a therapeutic agent is disposed on, in, or about at least a portion of an external surface of the first portion and is not disposed on, in or about the second portion. The therapeutic agent may be in or on a polymeric material. In some embodiments, the first portion of the device is configured or adapted to be implanted extravascularly and the second portion is configured or adapted to be implanted intravascularly. In some embodiments, the first portion of the device is configured or adapted to be implanted outside of a patient's CNS and the second portion is configured or adapted to be implanted within a patient's CNS. In some embodiments, a first therapeutic agent is disposed on, in, or about the first portion, and a second therapeutic agent is disposed on, about, or in the second portion. Such systems and devices, with therapeutic agents disposed on, in, or about specified portions configured to be implanted within certain tissues, allows for more targeted delivery of therapeutic agents and may increase efficacy and/or decrease undesired effects of the therapeutic agents.

It should be understood that, as used herein "implanted medical device", "implantable medical device", and the like refer to medical devices that are to be at least partially placed within a patient's body. Typically, such devices, or portions thereof, are placed within the patient's body for a period of time for which it would be beneficial to have a therapeutic agent present on an external surface of the device. For example, a medical device implanted in a patient's body for several hours or more constitutes an implantable medical device for the purposes of this disclosure.

### Systems and Devices

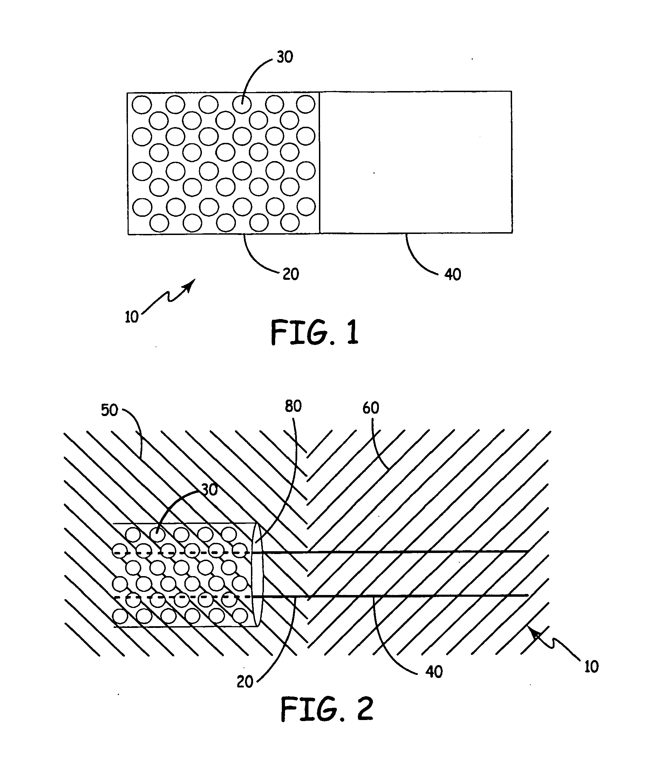

Referring to **Figure 1**, a device 10 comprising a first portion 20 adapted or configured to be implanted within a first tissue and a second portion 40 adapted or configured to be implanted within a second tissue is shown. A therapeutic agent 30 is disposed on, in, or about at least a portion of an exterior surface of the first portion 20 of the device 10. In some embodiments, the therapeutic agent 30 is capable of being released into the first tissue. In other embodiments, the therapeutic agent 30 may remain associated with device 10 and still provide its intended therapeutic action. It will be understood that the therapeutic agent need not be disposed on, in, or about the entire exterior surface of the first portion to produce a desirable effect. For example, the therapeutic agent may diffuse within the first tissue upon release from the polymeric material to provide a beneficial effect at a location removed from its site of release. Further, it may be desirable in some circumstances to release therapeutic agent only in limited areas of the first tissue.

Referring to **Figure 2**, a device 10 or system component is shown with a first portion 20 implanted in a first tissue 50 and a second portion 40 implanted in a second tissue 60. Therapeutic agent 30 is disposed on or in a polymeric material 80, which is disposed on or about an exterior surface of the first portion 20 of the device 10.

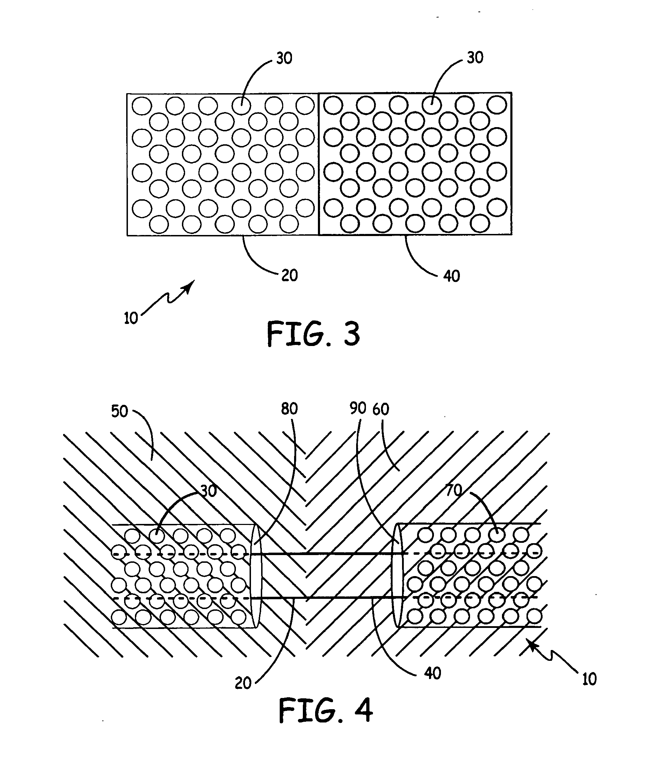

Referring to **Figure 3**, a device 10 having a first portion 20 adapted or configured to be implanted in a first tissue of a patient and a second portion 40 adapted or configured to be placed in a second tissue of a patient is shown. A first therapeutic agent 30 is disposed on, in, or about at least a portion of an external surface of the first portion 20 of the device. A therapeutic agent 70 is disposed on, in, or about at least a portion of an external portion of the second portion 40 of the device 10. **Figure 4** shows a device 10 comprising a first portion 20 and a second portion 40 implanted in a first tissue 50 and a second tissue 60; the first portion 20 of the device 10 being implanted in the first tissue and the second portion 40 of the device 10 being implanted in the second tissue 60. In the embodiment depicted in **Figure 4**, a first therapeutic agent 30 is disposed on or in a first polymeric material 80, which is disposed on or about the first portion of the device 10, and a second therapeutic agent 70 is disposed on or in a second polymeric material 90, which is disposed on or about the second portion 40 of the device 10. The first polymeric material 80 and second polymeric material 90 may be the same or different. The first therapeutic agent 30 and second therapeutic agent 70 may be the same, but may be present in or on the first polymeric material 30 and second polymeric material 70 in differing concentrations or amounts. Devices 10 configured as depicted in **Figures 3 and 4** allow for targeted delivery of different therapeutic agents or therapeutic agents of differing concentrations or amounts to different tissue.

Any catheter that is adapted or configured to be implanted in a patient in more than one tissue may be used in accordance with the teachings of this disclosure. Systems comprising such devices are also contemplated. Such systems include drug delivery systems, which include systems comprising implantable infusion pumps; neurostimulatory systems, which include systems comprising implantable pulse generators, such as spinal cord stimulation systems, deep brain stimulation systems, peripheral nerve stimulation systems, gastric stimulation systems, urological stimulation systems, and the like; and pacemaker and defibrillation systems.

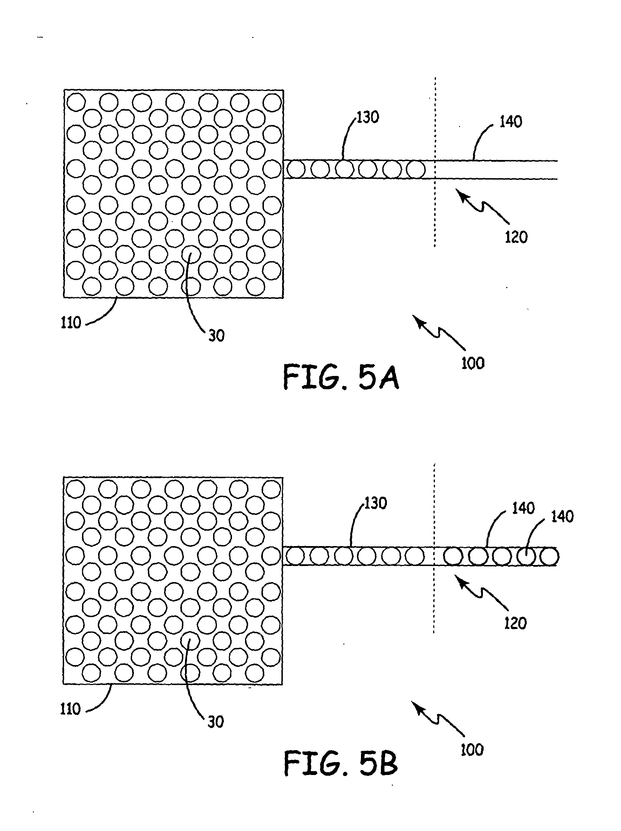

Referring to **Figure 5A**, a system 100 comprising an active device 110 and an associated device 120 is shown. Active device 110 may be, e.g., a pacemaker, defibrillator, pulse generator, infusion pump, and the like. It will be understood that "active" systems may operate through "passive" means. For example, implantable infusion pumps comprising expanded bladders that deliver fluid upon "passive" bellows or bladder contraction are considered active devices 110. Associated device 120 is a catheter.

Associated device 120 comprises a first portion 130 adapted or configured to be implanted in a first tissue and a second portion 140 adapted or configured to be placed in second tissue. In the embodiment shown in **Figure 5A**, a therapeutic agent 30 is disposed on, in, or about an external surface of first portion 130 of associated device 120 and an external surface of active device 110. It will be understood that a portion or the entire exterior surface of the first portion 130 of the associated device 120 and/or active device 110 may comprise a therapeutic agent 30 disposed therein, thereon, or thereabout. It will also be understood that active device 110, relative to first portion 130 of associated device 120, may have a different therapeutic agent disposed on, in, or about at least a portion of an external surface. It will be further understood that external surface of active device 120 or first portion 130 of associated device may, in some embodiments, comprise no therapeutic agent 30. Referring to **Figure 5B**, a second therapeutic agent 70 may be disposed about, on, or in at least a portion of an external surface of second, portion 140 of associated device 120.

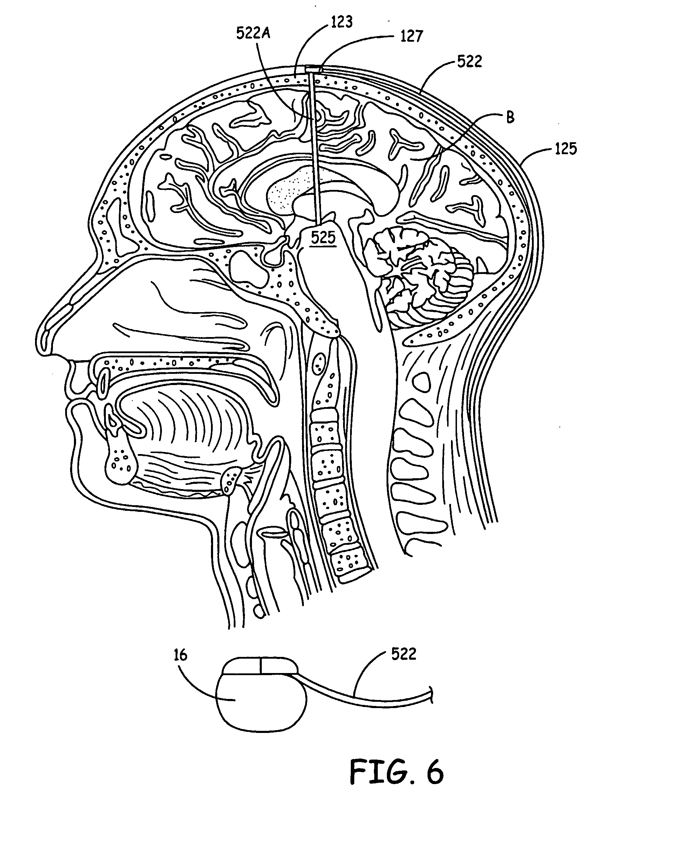

Figure 6 depicts a neurostimulator system implanted in a patient and is shown for background only. The system comprises an implantable pulse generator 16, a lead extension 522, a lead 522A, lead/lead extension connector 127, and at least one electrode positioned in proximity to the distal end of lead 522A. Pulse generator 16 is typically implanted subcutaneously in a patient, most typically in the abdomen or chest. However, it will be understood that pulse generator 16 may be implanted anywhere within a patient. Preferably the pulse generator 16 is implanted in a location that causes minimal discomfort to the patient and still allows for proper functioning. From the location of implantation of pulse generator 16, lead extension 522 is typically tunneled subcutaneously to a position in proximity to a target therapy site. In the embodiment shown in **Figure 6**, the target therapy site is within the patient's brain B. However, it will be understood that the target therapy site may be any other location where a patient may benefit from electrical stimulation therapy, such as e.g. other regions of the CNS and intravascular locations. Lead 522A is positioned such that one or more electrodes are in or in close proximity to the target therapy site. Lead 522A is typically connected to lead extension 522 through a connector 127. In the embodiment shown in Figure 6, a hole is drilled through the patient's skull 123 and lead 522A is inserted through the hole into patient's brain B such that one or more electrodes are in or near the target site.

By way of example, a therapeutic agent 30 may be disposed on, in, or about at least a portion of an external surface of one or more of pulse generator 16, lead extension 522, connector 127, and any other associated components (not shown), but not on, in, or about an external surface of lead 522A. Alternatively, a therapeutic agent 30 may be disposed on, in, or about at least a portion of an external surface of lead 522A, but not pulse generator 16, lead extension 522,or connector 127.

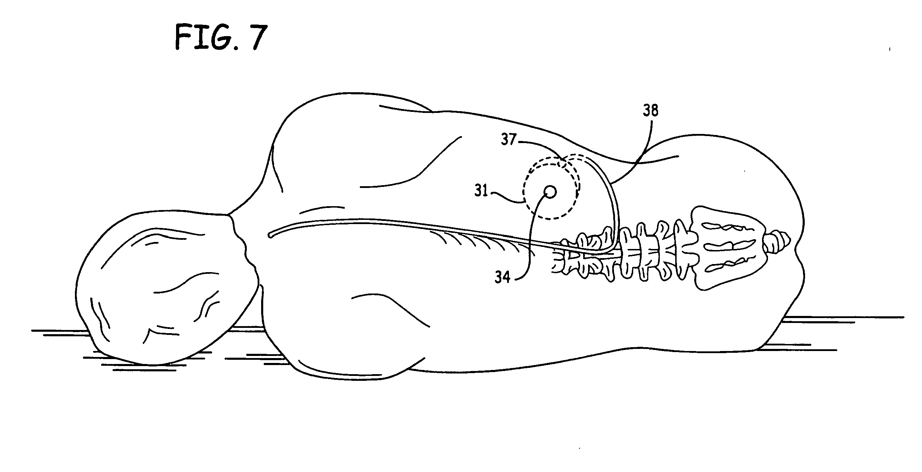

Referring to **Figure 7**, an infusion system implanted in a patient is shown. The infusion system comprises an implantable infusion pump 31 comprising a re-fill port 34 and a catheter connection port 37, and a catheter 38 connectable to the catheter connection port 37. Catheter comprises one or more infusion sites through which a drug housed in a reservoir of implantable pump 31 may be delivered to a target site of the patient. Typically, infusion pump 31 is implanted in a subcutaneous pocket in the patient as shown in **Figure 7**. The pump 31 may be implanted in any medically acceptable location within the patient. Typically, pump 31 is implanted into the patient's abdomen. The catheter is then typically tunneled to a location such that one or more infusion site is placed at or near a target treatment site in the patient. In **Figure 7**, the catheter 38 is introduced into the intrathecal space such that distal portion 39 of catheter resides within the patient's spinal column.

By way of example, a therapeutic agent 30 may be disposed on, in, or about at least a portion of an external surface of one or more of implantable infusion pump 31, an external surface of catheter 38 located outside patient's spinal canal, and any other associated components (not shown), but not on, in, or about an external surface of catheter 38 located within patient's spinal canal. Alternatively, therapeutic agent 30 may be disposed on, in, or about at least a portion of an external surface of catheter 38 located within a patient's spinal canal, but not a portion of catheter 38 located outside the patient's CNS or infusion pump 31. Additional combinations and alternatives are contemplated and may be readily derived.

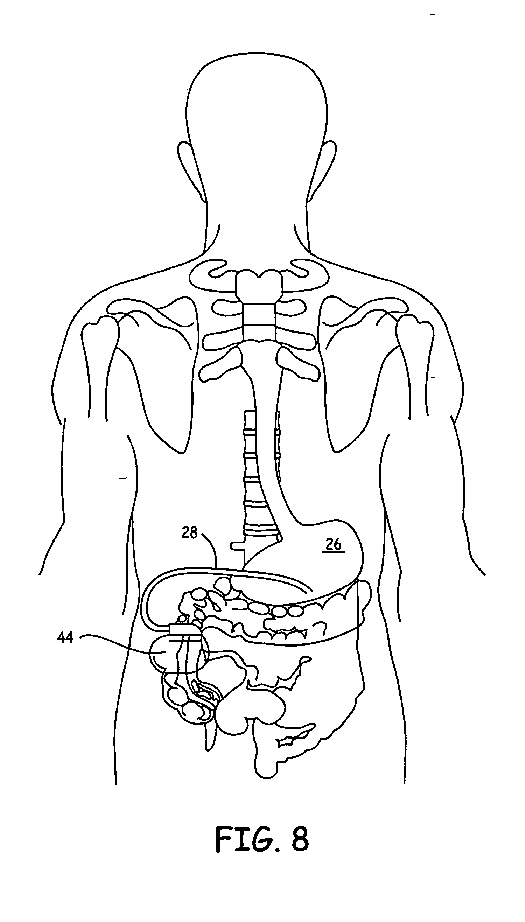

Referring to **Figure 8**, a gastric stimulation system is shown by way of example only. The system comprises an implantable pulse generator 44 and a lead 28 operably coupled to the pulse generator 44. The lead 28 comprises one or more electrodes (not shown) that are adapted or configured to stimulate the stomach 26. The lead 28 may be implanted in a position within the patient such that one or more of the electrodes are positioned to stimulate an enteric or autonomic nerve associated with the stomach 26. By way of example, a therapeutic agent 30 may be disposed about, on, or in at least a portion of lead 28 in proximity to the stomach 26 or enteric or autonomic nerve, but not portions of lead 28 not in proximity to the stomach 26 or enteric or autonomic nerve. Alternatively, a therapeutic agent 30, may be disposed about, on, or in at least a portion of the lead 28 not in proximity to the stomach 26 or enteric or autonomic nerve and pulse generator 44, but not a portion of lead 28 in proximity to stomach 26 or enteric or autonomic nerve.

### Tissue

Any medical device 10 adapted or configured to be implanted within more than one tissue location may be used in accordance with the teachings of this disclosure. By more than one tissue location, it is meant a tissue location into which it is desirable to introduce a medical device 10 having disposed therein, thereabout, or thereon a therapeutic agent 30, 70 and at least one other tissue location into which it would be less desirable to introduce a medical device 10 having disposed therein, thereabout, or thereon the same therapeutic agent 30, 70. For example, one tissue location may be extravascular tissue and another tissue location may be intravascular tissue; one tissue location may be non-CNS tissue and another may be CNS tissue; one tissue location may be subcutaneous tissue and another may be intravascular tissue or CNS tissue; one tissue location may be subcutaneous in an abdominal pocket and another may be subcutaneous in contact with or in proximity to a peripheral nerve; etc.

It should be understood that, as used herein, "tissue" includes fat and bodily fluids, such as blood and cerebrospinal fluid (CSF), with blood being an intravascular tissue and CSF being a CNS tissue.

Devices, or portions thereof, that are implanted in CNS tissue include devices implanted in intrathecal space, in epidural space, in intracerebroventricular space, and in the brain.

### Therapeutic Agent

Any therapeutic agent 30, 70 may be disposed on, in, or about device 10. Because it may be desirable to treat or prevent infections and/or inflammation associated with implantation of a medical device 10, it may be desirable to dispose one or more anti-infective agent and/or one or more anti-inflammatory agent in, on, or about at least a portion of an external surface of device 10. In addition, in some circumstances it may be desirable to deliver a local anesthetic to a location in proximity to a particular nerve or neuron or groups thereof, but not to particular subcutaneous regions removed from the particular nerves or neurons. Further in some circumstances, it may be desirable to deliver antiproliferative agents intravascularly, but nor extravascularly. As such, it may be desirable to provide a therapeutic agent 30 in, on, or about a portion of a device 10 or system 100 adapted or configured to be placed in proximity to a nerve or neuron, within the CNS, or intravascularly, but not to portions of the device 10 or system 100 configured or adapted to be placed away from the nerve or neuron, outside the CNS, or extravascularly.

### 1. Anti-infective Agents

Any anti-infective agent may be used in accordance with various embodiments of the invention. As used herein, "anti-infective agent" means an agent that kills or inhibits the growth of an infective organism, such as a microbe or a population of microbes. Anti-infective agents include antibiotics and antiseptics.

In an embodiment, an anti-infective agent is disposed in, on, or about at least a portion of a device 10 or system 100 implanted in subcutaneous tissue, but not in vascular or CNS tissue. In an embodiment, at least a portion of an active device 110 and/or an associated device 120 to be implanted in a subcutaneous tissue location has an anti-infective agent disposed thereon, therein, or thereabout, while portions to be implanted in CNS tissue or intravascularly do not. Because, the prevalence of infection associated with implantation of medical devices 10 or systems 100 is greatest in subcutaneous pockets, such configurations may be desirable.

### A. Antibiotic

Any antibiotic suitable for use in a human may be used in accordance with various embodiments of the invention. As used herein, "antibiotic" means an antibacterial agent. The antibacterial agent may have bateriostatic and/or bacteriocidal activities. Nonlimiting examples of classes of antibiotics that may be used include tetracyclines (e.g. minocycline), rifamycins (e.g. rifampin), macrolides (e.g. erythromycin), penicillins (e.g. nafcillin), cephalosporins (e.g. cefazolin), other beta-lactam antibiotics (e.g. imipenem, aztreonam), aminoglycosides (e.g. gentamicin), chloramphenicol, sufonamides (e.g. sulfamethoxazole), glycopeptides (e.g. vancomycin), quinolones (e.g. ciprofloxacin), fusidic acid, trimethoprim, metronidazole, clindamycin, mupirocin, polyenes (e.g. amphotericin B), azoles (e.g. fluconazole) and beta-lactam inhibitors (e.g. sulbactam). Nonlimiting examples of specific antibiotics that may be used include minocycline, rifampin, erythromycin, nafcillin, cefazolin, imipenem, aztreonam, gentamicin, sulfamethoxazole, vancomycin, ciprofloxacin, trimethoprim, metronidazole, clindamycin, teicoplanin, mupirocin, azithromycin, clarithromycin, ofloxacin, lomefloxacin, norfloxacin, nalidixic acid, sparfloxacin, pefloxacin, amifloxacin, enoxacin, fleroxacin, temafloxacin, tosufloxacin, clinafloxacin, sulbactam, clavulanic acid, amphotericin B, fluconazole, itraconazole, ketoconazole, and nystatin. Other examples of antibiotics, such as those listed in Sakamoto et al., U.S. Pat. No. 4,642,104, may also be used. One of ordinary skill in the art will recognize other antibiotics that may be used.

In general, it is desirable that the selected antibiotic(s) kill or inhibit the growth of one or more bacteria that are associated with infection following surgical implantation of a medical device. Such bacteria are recognized by those of ordinary skill in the art and include *Stapholcoccus aureus, Staphlococcus epidermis,* and *Escherichia coli.* Preferably, the antibiotic(s) selected are effective against strains of bacteria that are resistant to one or more antibiotic.

To enhance the likelihood that bacteria will be killed or inhibited, it may be desirable to combine two or more antibiotics. It may also be desirable to combine one or more antibiotic with one or more antiseptic. It will be recognized by one of ordinary skill in the art that antimicrobial agents having different mechanisms of action and/or different spectrums of action may be most effective in achieving such an effect. In an embodiment, a combination of rifampin and micocycline is used. In an embodiment, a combination of rifampin and clindamycin is used.

### B. Antiseptic

Any antiseptic suitable for use in a human may be used in accordance with various embodiments of the invention. As used herein, "antiseptic" means an agent capable of killing or inhibiting the growth of one or more of bacteria, fungi, or viruses. Antiseptic includes disinfectants. Nonlimiting examples of antiseptics include hexachlorophene, cationic bisiguanides (i.e. chlorhexidine, cyclohexidine) iodine and iodophores (i.e. povidone-iodine), para-chloro-meta-xylenol, triclosan, furan medical preparations (i.e. nitrofurantoin, nitrofurazone), methenamine, aldehydes (glutaraldehyde, formaldehyde), silver-containing compounds (silver sulfadiazene, silver metal, silver ion, silver nitrate, silver acetate, silver protein, silver lactate, silver picrate, silver sulfate), and alcohols. One of ordinary skill in the art will recognize other antiseptics that may be employed in accordance with this disclosure.

It is desirable that the antiseptic(s) selected kill or inhibit the growth of one or more microbe that are associated with infection following surgical implantation of a medical device. Such microbes are recognized by those of ordinary skill in the art and include *Stapholcoccus aureus, Staphlococcus epidermis, Escherichia coli, Pseudomonus auruginosa,* and *Candidia.*

To enhance the likelihood that microbes will be killed or inhibited, it may be desirable to combine two or more antiseptics. It may also be desirable to combine one or more antiseptics with one or more antibiotics. It will be recognized by one of ordinary skill in the art that antimicrobial agents having different mechanisms of action and/or different spectrums of action may be most effective in achieving such an effect. In a particular embodiment, a combination of chlorohexidine and silver sulfadiazine is used.

### 2. Anti-inflammatory Agents

Any anti-inflammatory agent suitable for use in a human may be used in accordance with various embodiments of the invention. Non-limiting examples of anti-inflammatory agents include steroids, such as prednisone, dexamethasone, and methyl-prednisilone; and non-steroidal anti-inflammatory agents (NSAIDs).

An embodiment of the invention provides devices 10 and systems 100 having an anti-inflammatory agent disposed on, in, or about at least a portion of the device 10 or system 100 to be implanted subcutaneously, but not intravascularly or in the CNS. Such configurations may be desirable to reduce systemic or CNS effect, while targeting the anti-inflammatory effects to subcutaneous locations susceptible to inflammation.

### 3. Local anesthetics

Any local anesthetic agent suitable for use in a human may be used in accordance with various embodiments of the invention. Non-limiting examples of local anesthetics agents include lidocaine, prilocaine, mepivicaine, bupivicaine and articaine.

An embodiment of the invention provides devices 10 and systems 100 having a local anesthetic agent disposed about, in, or, on at least a portion of the devices or systems to be implanted in proximity to a neuron or nerve, but not distant from the neuron or nerve. Such configurations may be desirable in situations where the device 10 or system 100 may impinge or rub a nerve. For example, leads associated with spinal cord stimulation have been known to impinge and/or rub nerves associated with the spinal cord causing pain. The direct targeting of local anesthetics to locations of possible nerve impingement or pain generation may be beneficial.

### 4. Anti-proliferative agents

Any local anti-proliferative agent suitable for use in a human may be used in accordance with various embodiments of the invention. As used herein, "anti-proliferative agents" includes anti-migration agents. In an embodiment, an anti-proliferative agent is an agent capable of preventing restenosis.

Examples of anti-proliferative agents include QP-2 (taxol), actinomycin, methotrexate, angiopeptin, vincristine, mitocycin, statins, C-MYC antisense, sirolimus, restenASE, 2-chloro-deoxyadenosine, PCNA (proliferating cell nuclear antigent) ribozyme, batimastat, prolyl hydroxylase inhibitors, halofuginone, C-proteinase inhibitors, probucol, and combinations and/or derivates thereof. In an embodinent, one or more anti-proliferative agent with one or more anti-inflammatory agent.

In an embodiment, at least a portion of a portion of a device 10 or system 100 to be implanted intravascularly, but not extravascularly, comprises an anti-proliferative agent disposed thereon, therein, or thereabout.

### 5. Association of Therapeutic Agent with Device

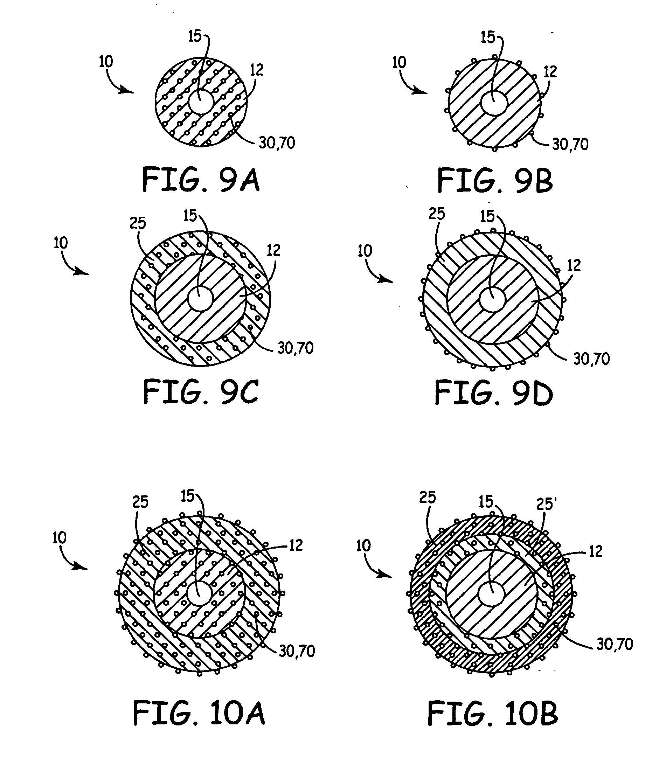

Therapeutic agent 30, 70 may be associated with a device 10 in any fashion such that contacting at least a portion of the device 10 with a tissue 50, 60 of a subject allows for the therapeutic agent 30, 70 to exert a therapeutic effect within the tissue 50, 60. **Figures 9A-9D** show examples of associations of therapeutic agent with device 10. **Figure 9A** shows that therapeutic agent 30, 70 may be disposed in a device 10. While **Figure 9A** shows therapeutic agent 30, 70 disposed throughout an external surface layer 12, the therapeutic agent 30, 70 may be disposed within one or more portions of the external surface layer 12 (not shown). **Figure 9B** shows that therapeutic agent 30, 70 may be disposed on the external surface layer 12. If a given therapeutic agent 30, 70 is disposed partially within the external surface 12 or other layer and partially protrudes from the surface layer 12 or other layer, the therapeutic agent 30, 70 is considered both disposed in and disposed on the external surface layer 12 or other layer. Further, while not shown, it will be understood that therapeutic agents 30, 70 may be both disposed in and disposed on the external surface layer 12 of the device 10. **Figures 9C and 9D** show embodiments where a coating layer 25 is disposed on the external surface layer 12 and the therapeutic agent 30, 70 is disposed in (**9C**) or on (**9D**) the coating layer. As with the external surface layer 12, therapeutic agent 30 may be disposed throughout the coating layer 25, in a portion of the coating layer 25, and/or both within and on the coating layer 25.

It will be understood that therapeutic agent 30, 70 as depicted in **Figures 10A-10D**, other subsequent Figures, and throughout the present disclosure may refer to a plurality of therapeutic agents 30, 70. For example, a therapeutic agent 30, 70 depicted in **Figure 10A** may be, *e.g*., minocycline and a different therapeutic agent 30, 70 may be, e.g., rifampin.

In various embodiments of the invention, therapeutic agents 30, 70 are disposed on, in, or about more than one layer of device 10. For example, therapeutic agent 30, 70 may be disposed on or in an external surface layer 12 of device 10 and/or on or in one or more coating layer 25 of device 10. **Figure 10A** shows an embodiment where therapeutic agent 30, 70 is disposed within or on external surface layer 12 and within or on coating layer 25 of device 10. **Figure 10B** shows an embodiment where therapeutic agent 30, 70 is disposed on or in a first coating layer 25 and on or in a second coating layer 25'. Of course, two, three, four, five, six, or more coating layers 25 may be disposed about external surface layer 12 of device 10 and therapeutic agent 30, 70 may be disposed in or on the external surface layer 12 and/or none, some, or all of the one or more coating layers 25.

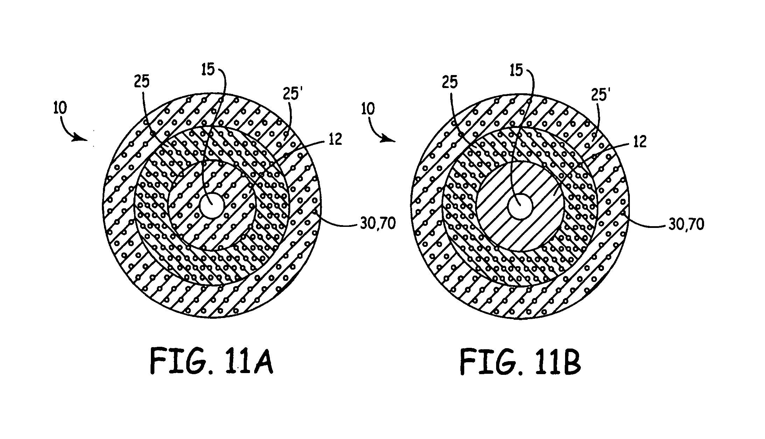

The concentration of therapeutic agents 30, 70 within various layers (depicted as external surface layer 12 or coating layer 25, 25') may be the same or different. Any concentration may be used. For example, therapeutic agent 30, 70 may comprise about 0.1% to about 50%, or from about 1% to about 10%, of the weight of the layer. In some circumstances, it may be desirable to place a higher concentration of therapeutic agent 30, 70 in one or more layers relative to other layers; e.g., when continued infusion of therapeutic agent 30, 70 into body tissue over time is desired. **Figure 11A** shows a device 10, where first coating layer 25 comprises a higher concentration of therapeutic agent 30, 70 within or on intermediate coating layer 25 than in outer coating layer 25' or external surface layer 12. In the embodiment illustrated by **Figure 11A**, external surface layer 12 is permeable to therapeutic agent 30, 70 and therapeutic agent 30, 70 may elute into lumen 15. Therapeutic agent 30, 70 may also elute out of outer coating layer 25' into body tissue. Increased initial concentration of therapeutic agent 30, 70 in intermediate coating layer 25 may effectively replenish the supply of therapeutic agent 30, 70 in outer coating layer 25' and body member 12 such that therapeutic agent 30, 70 may elute into lumen 15 or tissue. In the embodiment illustrated in **Figure 11B****,** external surface layer 12 is essentially impermeable to therapeutic agent 30, 70 and intermediate coating layer 25 comprises a higher concentration of therapeutic agent 30, 70 than outer coating layer 25'. Therapeutic agent 30, 70 in the intermediate coating layer may replenish supply in the outer coating layer 25' over time.

It should be understood that in certain embodiments of the invention, device 10 does not comprise a lumen 15.

It should be noted that coating layer 25, 25' as depicted in **Figures 9-11** may be polymeric material 80, 90 as depicted in **Figures 2** **and** **4**.

Release profile of therapeutic agent 30, 70 from device 10, may be varied. As described above, location of therapeutic agent 30, 70 in, on or about device 10, as well as concentration of therapeutic agent 30, 70 at a location, provides a means for achieving control over when therapeutic agent 30, 70 is released. The release profile may be varied by controlling the nature of the therapeutic agent 30, 70 to be released. For example, therapeutic agents 30, 70 having higher molecular weights would be expected to elute more slowly from device 10 than those having lower molecular weights. Thus, the extent to which a therapeutic agent 20 is hydrated may affect the rate at which therapeutic agent 30, 70 may elute out of device 10. Further, the extent to which therapeutic agent 30 interacts with external surface layer and/or other layers 25, 25'may affect the rate that therapeutic agent 30, 70 may elute out of device 10 into tissue. With these and other considerations in mind, it may be desirable, in some circumstances, to vary the location of slower eluting therapeutic agents 30, 70 and faster eluting therapeutic agents 30, 70 within, on, or about device 10.

The rate at which therapeutic agent 30, 70 may be released from device 10 into tissue may also be controlled by properties of coating layers 25 and/or external surface layer 12, as well as the manner in which therapeutic agent 30, 70 is disposed on or in coating layers 25 and/or external surface layer 12.

Any means of disposing a therapeutic agent 30, 70 on, in or about a device 10 or system 100 component may be used. For example, to dispose therapeutic agent 30, 70 in device 10, device 10 may be formed of a polymeric material into which therapeutic agent 30, 70 may be mixed prior to forming the device 10 or may be impregnated by, e.g. a solvent swelling technique, after device 10 has been formed. By way of further example, therapeutic agent 30, 70 may be disposed on the device 10 directly through chemical or physical means or may be incorporated into a polymeric material 80, 90 that is applied to device 10. By way of yet further example, therapeutic agent 30, 70 may be disposed about device 10 by being incorporated into a sheath, sleeve, jacket, cover, etc.

### Coating Layer

Coating layer 25, 25' may be formed of any material capable of releasing one or more therapeutic agent 30, 70 into tissue when placed in contact with the tissue. Preferably, coating layer 25, 25' is acceptable for at least temporary use within a human body. Coating layer 25, 25' is also preferably compatible with therapeutic agent 30, 70.

Examples of commonly used materials that may be used to form coating layers 25, 25' include organic polymers such as silicones, polyamines, polystyrene, polyurethane, acrylates, polysilanes, polysulfone, methoxysilanes, and the like. Other polymers that may be utilized include polyolefins, polyisobutylene and ethylene-alphaolefin copolymers; acrylic polymers and copolymers, ethylene-covinylacetate, polybutylmethacrylate; vinyl halide polymers and copolymers, such as polyvinyl chloride; polyvinyl ethers, such as polyvinyl methyl ether; polyvinylidene halides, such as polyvinylidene fluoride and polyvinylidene chloride; - polyacrylonitrile, polyvinyl ketones; polyvinyl aromatics, such as polystyrene, polyvinyl esters, such as polyvinyl acetate; copolymers of vinyl monomers with each other and olefins, such as ethylene-methyl methacrylate copolymers, acrylonitrile-styrene copolymers, ABS resins, and ethylene-vinyl acetate copolymers; polyamides, such as Nylon 66 and polycaprolactam; polycarbonates; polyoxymethylenes; polyimides; polyethers; epoxy resins; polyurethanes; rayon; rayon-triacetate; cellulose; cellulose acetate, cellulose butyrate; cellulose acetate butyrate; cellophane; cellulose nitrate; cellulose propionate; cellulose ethers; carboxymethyl cellulose; polyphenyleneoxide; and polytetrafluoroethylene (PTFE).

One or more coating layer 25, 25' according to various embodiments of the invention may comprise a biodegradable polymeric material, such as synthetic or natural bioabsorbable polymers. Synthetic bioabsorbable polymeric materials that can be used to form the coating layers include poly (L-lactic acid), polycaprolactone, poly(lactide-co-glycolide), poly(ethylene-vinyl acetate), poly(hydroxybutyrate-covalerate), polydioxanone, polyorthoester, polyanhydride, poly(glycolic acid), poly(D,L-lactic acid), poly(glycolic acid-co-trimethylene carbonate), polyphosphoester, polyphosphoester urethane, poly(amino acids), cyanoacrylates, poly(trimethylene carbonate), poly(iminocarbonate), copoly(ether-esters) such as PEO/PLA, polyalkylene oxalates, and polyphosphazenes. According to another exemplary embodiment of the present invention, the polymeric materials can be natural bioabsorbable polymers such as, but not limited to, fibrin, fibrinogen, cellulose, starch, collagen, and hyaluronic acid.

Coating layers 25, 25' may comprise polymeric materials designed to control the rate at which therapeutic agent is released, leached, or diffuses from the polymeric material. As used herein, "release", "leach", "diffuse", "elute" and the like are used interchangeably when referring to a therapeutic agent 30, 70 with respect to a coating layer 25 or external surface layer 12 of a device 10. Any known or developed technology may be used to control the release rate. For example, a coating layer may be designed according to the teachings of WO/04026361, entitled "Controllable Drug Releasing Gradient Coating for Medical Devices."

Preferable coating layer 25, 25' is formed from a non-biodegradable polymeric material. Preferably, coating layer 25, 25' is formed from silicone or polyurethane.

Coating layer 25, 25' of device 10 may be in the form of a tube, jacket, sheath, sleeve, cover, coating, or the like. Coating layer 25, 25' may be extruded, molded, coated on external surface layer 12, grafted onto external surface layer 12, embedded within external surface layer 12, adsorbed to external surface layer 12, etc. Polymers of coating layers 25, 25' may be porous or non-porous. Porous materials known in the art include those disclosed in U.S. Pat. No. 5,609,629 (Fearnot et al.) and U.S. Pat. No. 5,591,227 (Dinh et al.). Typically polymers are non-porous. However, non-porous polymers may be made porous through known or developed techniques, such as extruding with CO₂, by foaming the polymeric material prior to extrusion or coating, or introducing and then removing a porogen.

Depending upon the type of materials used to form coating layers 25, 25' of the present invention, the coatings can be applied to the external surface layer 12 or underlying coating layer 25, 25' through any coating processes known or developed in the art. One method includes directly bonding the coating material to a surface of body external surface layer 12 or underlying coating layer 25, 25'. By directly attaching a polymer coating to the external surface layer 12 or underlying coating layer 25, 25', covalent chemical bonding techniques may be utilized. External surface layer 12 or underlying coating layer 25, 25' surface may possess chemical functional groups on its surface such as carbonyl groups, primary amines, hydroxyl groups, or silane groups which will form strong, chemical bonds with similar groups on polymeric coating material utilized. In the absence of such chemical forming functional group, known techniques may be utilized to activate the material's surface before coupling the biological compound. Surface activation is a process of generating, or producing, reactive chemical functional groups using chemical or physical techniques such as, but not limited to, ionization, heating, photochemical activation, oxidizing acids, sintering, physical vapor deposition, chemical vapor deposition, and etching with strong organic solvents. Alternatively, the coating layer 25, 25' may be indirectly bound to body member 12 or underlying coating layer 25, 25' through intermolecular attractions such as ionic or Van der Waals forces. Of course, if coating layer 25, 25' is in the form of a jacket, sheath, sleeve, cover, or the like the chemical interaction between coating layer 25, 25' and external surface layer 12 may be minimal.

Therapeutic agent 30 may be incorporated into a coating layer 25, 25' in a variety of ways. For example, therapeutic agent 30 can be covalently grafted to a polymer of the coating layer 25, 25', either alone or with a surface graft polymer. Alternatively, therapeutic agent 30 may be coated onto the surface of the polymer either alone or intermixed with an overcoating polymer. Therapeutic agent 30 may be physically blended with a polymer of a coating layer 25, 25' as in a solid-solid solution. Therapeutic agent 30 may be impregnated into a polymer by swelling the polymer in a solution of the appropriate solvent. Any means of incorporating therapeutic agent 30 into or on a coating layer 25, 25' may be used, provided that therapeutic agent 30 may be released, leached or diffuse from coating layer 25, 25' on contact with bodily fluid or tissue.

A polymer of a coating layer 25, 25' and a therapeutic agent 30 may be intimately mixed either by blending or using a solvent in which they are both soluble. This mixture can then be formed into the desired shape or coated onto an underlying structure of the medical device. One exemplary method includes adding one or more therapeutic agent 30 to a solvated polymer to form a therapeutic agent 30/polymer solution. The therapeutic agent 30/polymer solution can then be applied directly to the external surface layer 12 or underlying coating layer 25, 25'; for example, by either spraying or dip coating device 10. As the solvent dries or evaporates, the therapeutic agent 30/polymer coating is deposited on device 10. Furthermore, multiple applications can be used to ensure that the coating is generally uniform and a sufficient amount of therapeutic agent 30 has been applied to device 10.

Alternatively, an overcoating polymer, which may or may not be the same polymer that forms the primary polymer of external surface layer 12 (it will be understood that in some embodiments the external surface layer 12 of device 10 is formed of a polymeric material and in other embodiments the external surface layer 12 of device 10 is from non-polymeric material, such as metallic material) or underling coating layer 25, 25', and therapeutic agent 30 are intimately mixed, either by blending or using a solvent in which they are both soluble, and coated onto external surface layer 12 or underling coating layer 25, 25'. Any overcoating polymer may be used, as long as the polymer is able to bond (either chemically or physically) to the polymer of an underlying layer of device 10.

In addition, a polymer of a coating layer 25, 25' may be swelled with an appropriate solvent, allowing a therapeutic agent 30, 70 to impregnate the polymer.

Therapeutic agent 30, 70 may also be covalently grafted onto a polymer of a coating layer 25, 25'. This can be done with or without a surface graft polymer. Surface grafting can be initiated by corona discharge, UV irradiation, and ionizing radiation. Alternatively, the ceric ion method, previously disclosed in U.S. Pat. No. 5,229,172 (Cahalan et al.), may be used to initiate surface grafting.

## Claims

1. An implantable medical catheter (10), comprising an external surface, the external surface of the catheter comprising,
(a) a first portion (20) adapted to be implanted into a first tissue location and
(b) a second portion (40) adapted to be implanted into a second tissue location; and
a first therapeutic agent (30) disposed on, in, or about at least a portion of the first portion but not the second portion.

2. The catheter of claim 1, further comprising a coating layer disposed on or about the at least a portion of the first portion, wherein the first therapeutic agent is disposed in the coating layer.

3. The catheter of claim 2, wherein the coating layer comprises a polymeric material.

4. The catheter of claim 2, wherein the coating layer is in the form of a sheath, sleeve, jacket or cover.

5. A system comprising a first medical device (110) having an external surface, and an implantable medical catheter as claimed in any preceding claim, configured to be coupled to said first medical device.

6. A system as claimed in claim 5, wherein said first medical device is an implantable infusion pump (31).

## Patentansprüche

1. Implantierbarer medizinischer Katheter (10), umfassend eine Außenoberfläche, wobei die Außenoberfläche des Katheters Folgendes umfasst:
(a) einen ersten Teil (20), der dafür ausgelegt ist, an einem ersten Gewebeort implantiert zu werden und
(b) einen zweiten Teil (40), der dafür ausgelegt ist, an einem zweiten Gewebeort implantiert zu werden; und
einen ersten therapeutischen Wirkstoff (30), der auf bzw. in zumindest einem Teil des ersten, aber nicht des zweiten Teils, oder um diesen herum, angeordnet ist.

2. Katheter nach Anspruch 1, des Weiteren umfassend eine Überzugsschicht, die auf zumindest einem Teil des ersten Teils, oder um diesen herum, angeordnet ist, wobei der erste therapeutische Wirkstoff in der Überzugsschicht angeordnet ist.

3. Katheter nach Anspruch 2, wobei die Überzugsschicht ein polymeres Material umfasst.

4. Katheter nach Anspruch 2, wobei die Überzugsschicht in Form einer Scheide, einer Hülle, eines Umschlags oder einer Abdeckung vorliegt.

5. System, umfassend eine erste medizinische Vorrichtung (110) mit einer Außenoberfläche, und implantierbarer medizinischer Katheter, wie in einem der vorangehenden Ansprüche beansprucht, welcher derart konfiguriert ist, dass er an die erste medizinische Vorrichtung gekoppelt ist.

6. System, wie in Anspruch 5 beansprucht, wobei die erste medizinische Vorrichtung eine implantierbare Infusionspumpe (31) ist.

## Revendications

1. - Cathéter médical implantable (10), comprenant une surface externe, la surface externe du cathéter comprenant :
(a) une première partie (20) apte à être implantée dans un premier emplacement de tissu et
(b) une seconde partie (40) apte à être implantée dans un second emplacement de tissu ; et
un premier agent thérapeutique (30) disposé sur, dans ou autour d'au moins une partie de la première partie mais pas de la seconde partie.

2. - Cathéter selon la revendication 1, comprenant en outre une couche de revêtement disposée sur ou autour d'au moins une partie de la première partie, le premier agent thérapeutique étant disposé dans la couche de revêtement.

3. - Cathéter selon la revendication 2, dans lequel la couche de revêtement comprend une matière polymère.

4. - Cathéter selon la revendication 2, dans lequel la couche de revêtement est sous la forme de gaine, de manchon, de chemise ou d'enveloppe.

5. - Système comprenant un premier dispositif médical (110) ayant une surface externe, et un cathéter médical implantable tel que revendiqué à l'une quelconque des revendications précédentes, configuré pour être couplé audit premier dispositif médical.

6. - Système selon la revendication 5, dans lequel ledit premier dispositif médical implantable est une pompe à perfusion implantable (31).
